# EUROPEAN PATENT APPLICATION

(11) **EP 4 749 264 A1**
(43) Date of publication of application: **27.05.2026**
(21) Application number: 24845356.5
(22) Date of filing: 04.07.2024
(51) Int. Cl.: G01N 21/01, A61B 5/00, G01N 21/27, H10H 20/00, H10H 20/851

(54) **NEAR-INFRARED LIGHT EMITTING DEVICE AND BIOLOGICAL INFORMATION DETECTION DEVICE PROVIDED WITH SAME**

(30) Priority: 21.07.2023 JP 2023119019
(71) Applicant: Panasonic Intellectual Property Management Co., Ltd., Kadoma-shi, Osaka 571-0057 (JP)
(72) Inventor: NITTA, Mitsuru, Kadoma-shi, Osaka 571-0057 (JP); OSHIO, Shozo, Kadoma-shi, Osaka 571-0057 (JP); IWATA, Koki, Kadoma-shi, Osaka 571-0057 (JP); FUJIWARA, Chigusa, Kadoma-shi, Osaka 571-0057 (JP)
(74) Representative: Müller-Boré & Partner Patentanwälte PartG mbB
(86) International application number: PCT/JP2024/024268
(87) International publication number: WO 2025/022975

(57) **Abstract**

Provided is a near-infrared light emitting device (10) including: a solid-state light emitting element (1); and a wavelength converter (2) that includes a phosphor, wherein the near-infrared light emitting device emits two or more fluorescent components from one light output surface. The two or more fluorescent components include a first fluorescent component and a second fluorescent component positioned in order from a short wavelength side. In a spectral shape of the first fluorescent component, a slope in a long wavelength region from a fluorescence peak of the first fluorescent component is steeper than that in a short wavelength region, and in a spectral shape of the second fluorescent component, a slope in a short wavelength region from a fluorescence peak of the second fluorescent component is steeper than that in a long wavelength region. The second fluorescent component has the fluorescence peak, which is within a wavelength range of 780 nm or more and 1,000 nm or less.

## Description

### TECHNICAL FIELD

The present disclosure relates to a near-infrared light emitting device and a biological information detection device provided with the near-infrared light emitting device.

### BACKGROUND ART

In the fields of biochemistry, medicine, agriculture, nutrition, and the like, a two wavelength method (dual wavelength spectrophotometry) has been conventionally used in order to detect a predetermined substance even when it is difficult to purely separate a sample. The two wavelength method is also used to identify substances added to various foods, spoilage products, and substances added to various pharmaceuticals.

The two wavelength method is a method of measuring the absorbance of a first wavelength that is a maximum absorption wavelength, and the absorbance of a second wavelength set at a longer wavelength side, and then quantifying a concentration of a target substance from the difference in absorbance between a primary wavelength and a secondary wavelength. For example, a pulse oximeter uses two types of light, red and infrared, to determine the ratio of hemoglobin bound to oxygen among hemoglobin in blood.

As a device for detecting biological information using such a two wavelength method, a device disclosed in Patent Literature 1 is known. Patent Literature 1 discloses a biological information detection device for detecting biological information on blood, such as blood oxygen saturation, or hemoglobin concentration, in a non-contact manner. The biological information detection device includes a light source for irradiating the living body, which is a measurement object, with measurement light of multiple different wavelengths, and an irradiation angle adjusting unit as an irradiation position adjusting unit for adjusting an irradiation position of the light of multiple different wavelengths according to a measurement distance to the measurement object. The biological information detection device further includes an imaging unit as a light receiving unit for receiving reflected light from the living body which is a measurement object, and a processing unit for calculating biological information, based on intensity of the reflected light. With the biological information detection device, biological information is detected in a non-contact manner by irradiating a subject with measurement light of multiple different wavelengths at the same irradiation position, and calculating the blood oxygen saturation or hemoglobin concentration, based on the intensity of reflected light of the measurement light at the irradiation position.

### CITATION LIST

### PATENT LITERATURE

Patent Literature 1: Japanese Patent No. 6832512

### SUMMARY OF INVENTION

In the two wavelength method, two or more light sources are usually used when light of two different wavelengths is emitted. However, when multiple light sources of different wavelengths are arranged, since optical axes of the light sources are different, there is a case where irradiation unevenness occurs in light emitted on a measurement object, and the intensity balance differs depending on irradiation positions. If irradiation unevenness occurs in light emitted on the measurement object, there is a possibility that measurement results are adversely affected.

The present invention has been made in consideration of the above issues, which are inherent in the related art. An object of the present invention is to provide a near-infrared light emitting device capable of controlling the occurrence of irradiation unevenness in light emitted on a measurement object in a case of irradiating with light of two different wavelengths, and a biological information detection device provided with the near-infrared light emitting device.

In response to the above issues, a near-infrared light emitting device according to a first aspect of the present invention includes: a solid-state light emitting element; and a wavelength converter that includes a phosphor, wherein the near-infrared light emitting device emits two or more fluorescent components from one light output surface. The two or more fluorescent components include a first fluorescent component and a second fluorescent component positioned in order from a short wavelength side, the first fluorescent component and the second fluorescent component being based on electron energy transition of a transition metal ion. In a spectral shape of the first fluorescent component, a slope in a long wavelength region from a fluorescence peak of the first fluorescent component is steeper than that in a short wavelength region. In a spectral shape of the second fluorescent component, a slope in a short wavelength region from a fluorescence peak of the second fluorescent component is steeper than that in a long wavelength region. An emission intensity of the first fluorescent component at a long wavelength end is 10% of a maximum emission intensity at the fluorescence peak of the first fluorescent component and at the fluorescence peak of the second fluorescent component, and an emission intensity of the second fluorescent component at a short wavelength end is 10% of the maximum emission intensity. The second fluorescent component has the fluorescence peak, which is within a wavelength range of 780 nm or more and 1,000 nm or less.

A near-infrared light emitting device according to a second aspect of the present invention includes: a solid-state light emitting element; and a wavelength converter that includes a phosphor, wherein the near-infrared light emitting device emits two or more fluorescent components from one light output surface. The near-infrared light emitting device further includes a notch filter for controlling wavelength-converted light converted by the wavelength converter. The fluorescent components include a first fluorescent component on a short wavelength side, and a second fluorescent component on a long wavelength side across a blocking region controlled by the notch filter. The first fluorescent component and the second fluorescent component are based on electron energy transition of a transition metal ion. The second fluorescent component has a fluorescence peak within a wavelength range of 780 nm or more and 1,000 nm or less.

A biological information detection device according to a third aspect of the present invention includes a near-infrared light emitting device.

### BRIEF DESCRIPTION OF DRAWINGS

[Fig. 1] Fig. 1 is a diagram illustrating the relationship between an irradiation position of a measurement object and an emission spectrum of irradiation light when two types of light emitting diodes having different wavelengths of irradiation light are used as a light source for a two wavelength method.
[Fig. 2] Fig. 2 is a diagram illustrating the relationship between an irradiation position of a measurement object, and an emission spectrum of irradiation light when a light emitting device having a near-infrared phosphor is used as a light source for the two wavelength method.
[Fig. 3] Fig. 3(a) is a schematic diagram illustrating an example configuration of a near-infrared light emitting device according to the present embodiment. Fig. 3(b) is a diagram illustrating an example of a spectral distribution of output light emitted from the near-infrared light emitting device according to the present embodiment. Fig. 3(c) is a diagram illustrating an example of a spectral distribution of wavelength-converted light emitted from a wavelength converter before passing through a notch filter in the near-infrared light emitting device according to the present embodiment.
[Fig. 4] Fig. 4 is a diagram illustrating an example of a spectral distribution of output light emitted from the near-infrared light emitting device according to the present embodiment.
[Fig. 5] Fig. 5 is a schematic diagram illustrating an example of a biological information detection device according to the present embodiment.

### DESCRIPTION OF EMBODIMENTS

Referring to the drawings, a description is given below of a near-infrared light emitting device according to the present embodiment, and a biological information detection device provided with the near-infrared light emitting device. Note that dimensional ratios in the drawings are exaggerated for convenience of the description and are sometimes different from actual ratios.

### [Near-infrared light emitting device]

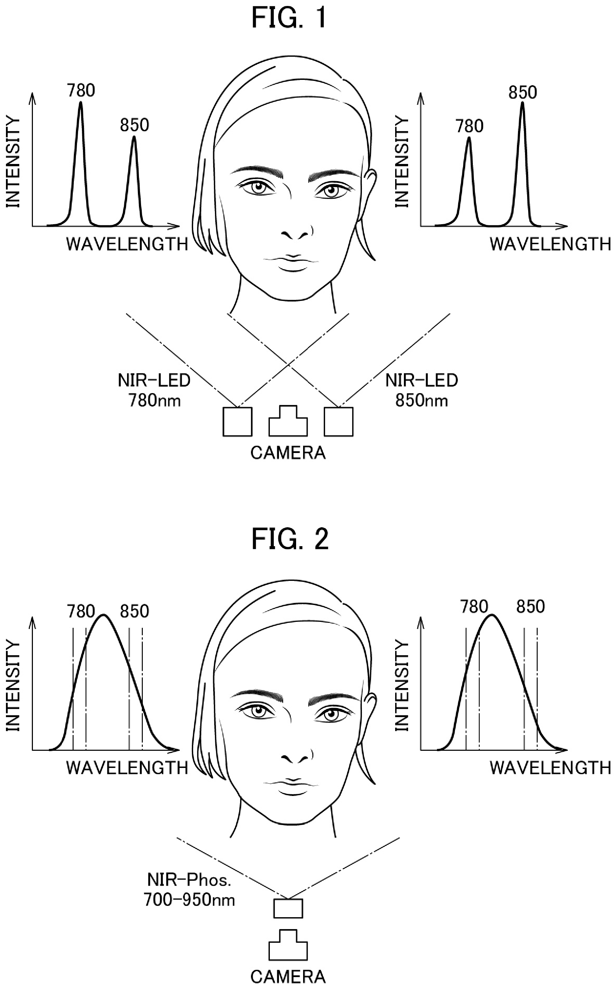

In the two wavelength method, at least two light sources are generally used when emitting light of two different wavelengths. For example, as illustrated in Fig. 1, a light emitting device is used where two types of light emitting diodes (LED) having different output light wavelengths are arranged in parallel relative to a measurement object. An imaging camera that detects reflected light from the measurement object is arranged between the two types of light emitting diodes. In this light emitting device, one of the light emitting diodes is a near-infrared light emitting diode and emits output light having a peak wavelength of 780 nm. The other of the light emitting diodes is also a near-infrared light emitting diode and emits output light having a peak wavelength of 850 nm. The light emitting diodes each emit sharp output light having a small half width.

When near-infrared light is emitted on the face of a person, which is a measurement object, using the light emitting device illustrated in Fig. 1, both near-infrared light having a wavelength of 780 nm, and near-infrared light having a wavelength of 850 nm are emitted on the face of the person. Here, in light emitted on the left half of the face of the person, the irradiation intensity of the near-infrared light having a wavelength of 780 nm is greater than that of the near-infrared light having a wavelength of 850 nm. On the other hand, in light emitted on the right half of the face of the person, the irradiation intensity of the near-infrared light having a wavelength of 850 nm is greater than that of the near-infrared light having a wavelength of 780 nm. In this way, when multiple light sources having different wavelengths are arranged, since optical axes of the light sources are different from each other, irradiation unevenness occurs in the near-infrared light emitted on the face of the person, and a phenomenon occurs where the intensity balance differs depending on irradiation locations. In addition, since temperature features of these two types of light emitting diodes also differ from each other, there is an issue that the irradiation intensity balance changes depending on surrounding ambient temperature.

In order to control such irradiation unevenness relative to a measurement object, instead of the LED system illustrated in Fig. 1, a phosphor system illustrated in Fig. 2 is attracting attention. A light emitting device illustrated in Fig. 2 is provided with a solid-state light emitting element, and a wavelength converter including a near-infrared phosphor, and after wavelength conversion of primary light emitted from the solid-state light emitting element using the near-infrared phosphor, near-infrared light is emitted from a light output surface. An imaging camera for detecting reflected light from a measurement object is arranged in the vicinity of the light emitting device. The near-infrared light emitted from the light emitting device is broad fluorescence having a large half width. By using two fluorescent components of the broad fluorescence, a target substance can be quantified. For example, in the light emitting device of Fig. 2, a target substance is quantified by using a fluorescent component having a wavelength around 780 nm, and a fluorescent component having a wavelength around 850 nm.

Here, when near-infrared light is emitted on the face of a person, which is a measurement object, using the light emitting device illustrated in Fig. 2, both the left half and the right half of the face of the person are irradiated with near-infrared light having a constant intensity balance. That is, in the light emitting device illustrated in Fig. 2, near-infrared light is emitted from one light output surface, and thus the optical axis is one. Even when there are multiple solid-state light emitting elements and wavelength converters, the light output surface from which near-infrared light is emitted is one, and thus the optical axes are the same. Thus, since the intensity balance of near-infrared light is constant at any position of the measurement object, the target substance can be quantified with high accuracy by detecting reflected light of two wavelengths from the measurement object. The light emitting device illustrated in Fig. 2 uses a wavelength converter including a near-infrared phosphor, and since temperature properties of the wavelength converter are constant, the intensity balance of irradiation light does not easily change even if surrounding ambient temperature changes.

As described above, unlike the LED type light emitting device illustrated in Fig. 1, the phosphor type light emitting device illustrated in Fig. 2 is characterized in that the optical axes of near-infrared light emitted on a measurement object are the same, and the intensity balance is constant at any position of the measurement object.

Here, in the LED type light emitting device illustrated in Fig. 1 and the phosphor type light emitting device illustrated in Fig. 2, for example, light having a wavelength of around 780 nm, and light having a wavelength of around 850 nm are emitted. In addition, in the light emitting device illustrated in Fig. 2, fluorescence between a wavelength of 780 nm and a wavelength of 850 nm, that is, fluorescence in a wavelength range of 790 nm to 840 nm is also emitted. Furthermore, since the near-infrared light emitted from the light emitting device of Fig. 2 has a fluorescence peak in the wavelength range of 790 nm to 840 nm, fluorescence having the wavelength of 790 nm to 840 nm has a high intensity.

When such near-infrared light having a high intensity, which is not used in quantification with the two wavelength method, is emitted on a person who is a measurement object, for example, there is a possibility of giving discomfort due to glare, and/or thermal discomfort. Thus, when the measurement object is irradiated with near-infrared fluorescence, it is preferable to exclude unnecessary light having a wavelength other than the two wavelengths used for the quantitative measurement.

The light emitting device according to the present embodiment is a device capable of controlling irradiation of unnecessary fluorescence while controlling irradiation unevenness in light emitted on a measurement object, even when light of two different wavelengths is emitted.

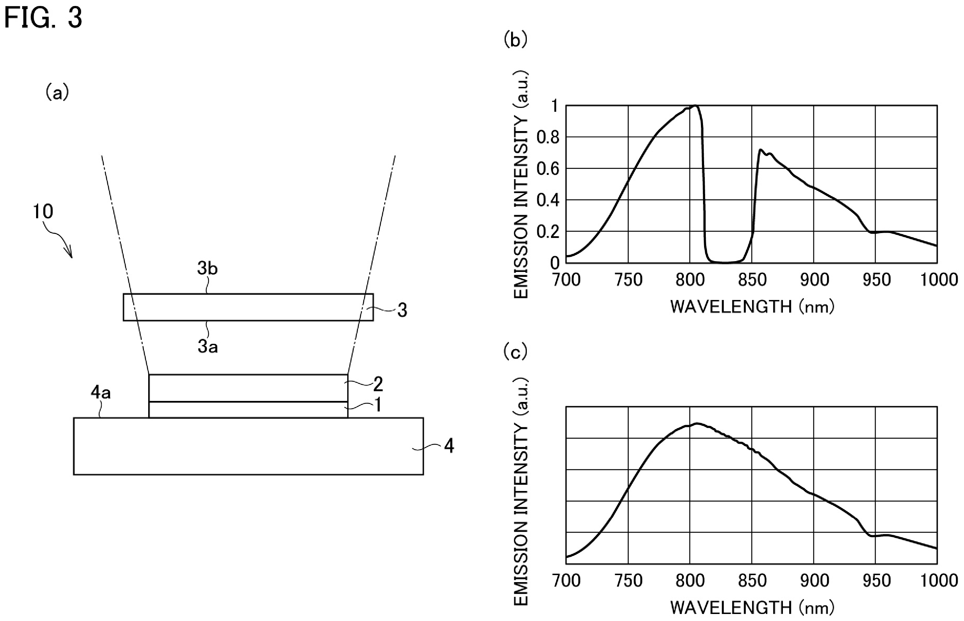

As illustrated in Fig. 3(a), a near-infrared light emitting device 10 according to the present embodiment includes a solid-state light emitting element 1 that emits primary light, and a wavelength converter 2 that includes a phosphor having a property of absorbing the primary light and converting it into an optical component having a wavelength longer than that of the primary light. The near-infrared light emitting device 10 further includes an optical member 3 that attenuates part of fluorescence emitted by the phosphor included in the wavelength converter 2 but transmits other fluorescence.

The solid-state light emitting element 1 is mounted on a surface (main surface) 4a of a substrate 4. For the solid-state light emitting element 1, a light emitting diode (LED) or a laser diode can be used, for example. By using an LED module or a laser diode emitting high energy light of 1 W or more, for example, a near-infrared light emitting device can be expected to achieve a light output in the class of several hundred mW. By using an LED module emitting high energy light of 3 W or more, or 10 W or more, a near-infrared light emitting device can be expected to achieve a light output in the class of several watts. By using an LED module emitting high energy light of 30 W or more, a near-infrared light emitting device can be expected to achieve a light output in the class of more than 10 W. By using an LED module emitting high energy light of 100 W or more, a near-infrared light emitting device can be expected to achieve a light output in the class of more than 30 W.

When a laser diode is used as the solid-state light emitting element 1, and primary light is set as laser light, a specification is made where the wavelength converter 2 is irradiated with a high-density spotlight. Hence, the near-infrared light emitting device obtained can be a point light source of high output, and thus a range of industrial use of solid state lighting can be expanded. For example, an edge emitting laser (EEL), a vertical cavity surface emitting laser (VCSEL), or the like can be used as the laser diode.

The primary light emitted by the solid-state light emitting element 1 is preferably blue light having a maximum intensity within a wavelength range of 435 nm or more, and less than 480 nm. Since the solid-state light emitting element, which emits high-output and high-efficiency blue light, is easy to procure, it is easy to achieve higher output and higher efficiency of the near-infrared light emitting device, and furthermore, it is advantageous for the industrial production of the near-infrared light emitting device.

Note that the number of the solid-state light emitting elements 1 mounted on the substrate 4 is not particularly limited, and may be a single or multiple. By using multiple solid-state light emitting elements 1, the output of primary light can be easily increased, and thus the near-infrared light emitting device is advantageous for high output. The number of solid-state light emitting elements is not particularly limited, but may be appropriately selected from, for example, 9 or more, 16 or more, 25 or more, 36 or more, 49 or more, 64 or more, 81 or more, or 100 or more. The upper limit of the number is not particularly limited, but may be appropriately selected from, for example, 9 or less, 16 or less, 25 or less, 36 or less, 49 or less, 64 or less, 81 or less, or 100 or less.

In the near-infrared light emitting device 10, the solid-state light emitting element 1 is preferably a surface-emitting light source of a surface-emitting type. This controls variations in intensity distribution and unevenness in color tone of primary light with which the wavelength converter 2 is irradiated, and thus the near-infrared light emitting device is advantageous in controlling unevenness in intensity distribution of output light.

A material of the substrate 4 is not particularly limited, but for example, a substrate made from a metal can be used, and specifically, a substrate made from aluminum can be used. As the substrate 4, for example, a substrate made from a ceramic can be used, and specifically, a substrate made from alumina can be used.

The wavelength converter 2 is stacked on the upper surface of the solid-state light emitting element 1. The wavelength converter 2 preferably contains a near-infrared phosphor. The near-infrared phosphor is a phosphor that absorbs primary light emitted by the solid-state light emitting element 1 and converts it into wavelength-converted light including near-infrared light. Such a near-infrared phosphor is preferably a phosphor that emits near-infrared light having a fluorescence peak within a wavelength range of 730 nm or more and less than 2,500 nm, and preferably a phosphor that emits near-infrared light having a fluorescence peak within a wavelength range of 780 nm or more and less than 2,500 nm. The near-infrared phosphor is preferably a phosphor that emits near-infrared light having a fluorescence peak within a wavelength range of 730 nm or more and less than 1,000 nm, and more preferably a phosphor that emits near-infrared light having a maximum intensity within a wavelength range of 780 nm or more and less than 1,000 nm. Thus, since primary light emitted from a solid-state light emitting element can be easily wavelength-converted into a near-infrared optical component, it is advantageous, for example, to obtain a near-infrared optical component required for measurement light.

As the near-infrared phosphor, for example, various inorganic phosphors known for near-infrared light sources can be used. Specifically, as the near-infrared phosphor, a phosphor that is activated with at least one of a transition metal ion or a rare earth ion, and emits fluorescence including a near-infrared optical component can be used. The transition metal ion is preferably at least one selected from the group consisting of Ti³⁺, V⁴⁺, Cr⁴⁺, V³⁺, Cr³⁺, V²⁺, Mn⁴⁺, Fe³⁺, Co³⁺, Co²⁺, and Ni²⁺. The rare earth ion is preferably at least one selected from the group consisting of Nd³⁺, Eu²⁺, Ho³⁺, Er³⁺, Tm³⁺, and Yb³⁺. The near-infrared phosphor is preferably an oxide, sulfide, nitride, halide, oxysulfide, oxynitride, or oxyhalide, including the fluorescent ion.

The near-infrared phosphor included in the wavelength converter 2 can be at least one selected from the group consisting of a halophosphate, a phosphate, a halosilicate, a silicate, an aluminate, an aluminosilicate, a borate, a germanate, a silicate nitride, an aluminosilicate nitride, a silicate oxynitride, and an aluminosilicate oxynitride, which are activated by an activator described above. Thus, a suitable phosphor for lighting design may be appropriately selected and used from these.

Here, the activator of the near-infrared phosphor included in the wavelength converter 2, that is, the fluorescent ion, is preferably Cr³⁺. By using Cr³⁺, it is possible to obtain a phosphor having a property of absorbing visible light, especially blue light or red light, and converting it into deep-red to near-infrared optical components. In addition, depending on the type of host to which an activator is added, it becomes easy to change a light absorption peak wavelength or a fluorescence peak wavelength of a phosphor, and this is advantageous in changing an excitation spectral shape or a fluorescence spectral shape. Furthermore, many Cr³⁺ activated phosphors that absorb blue light or red light and convert it into a near-infrared fluorescent component are also known. This not only broadens selection of the solid-state light emitting element 1, which emits primary light, but also makes it easier to change the peak wavelength of fluorescence emitted by the phosphor, and thus provides the near-infrared light emitting device 10, which is advantageous in controlling the spectral distribution of output light.

Note that a phosphor where the fluorescent ion is Cr³⁺ is not particularly limited as long as it absorbs excitation light (primary light) and converts it into fluorescence having a wavelength long than that of the excitation light, and can be appropriately selected from known Cr³⁺ activated phosphors. However, it is preferable that the Cr³⁺ activated phosphor be based on a composite metal oxide, which is easy to manufacture.

It is preferable that the Cr³⁺ activated phosphor be a composite oxide phosphor having a garnet-type crystal structure, which has many practical applications. Such a Cr³⁺ activated garnet phosphor can be at least one selected from the group consisting of Y₃Al₂(AlO₄)₃:Cr³⁺, La₃Al₂(AlO₄)₃:Cr³⁺, Gd₃Al₂(AlO₄)₃:Cr³⁺, Y₃Ga₂(AlO₄)₃:Cr³⁺, La₃Ga₂(AlO₄)₃:Cr³⁺, Gd₃Ga₂(AlO₄)₃:Cr³⁺, Y₃Sc₂ (AlO₄)₃:Cr³⁺, La₃Sc₂(AlO₄)₃:Cr³⁺, Gd₃Sc₂(AlO₄)₃:Cr³⁺ Y₃Ga₂(GaO₄)₃:Cr³⁺, La₃Ga₂(GaO₄)₃:Cr³⁺, (Gd, La)₃Ga₂(GaO₄)₃:Cr³⁺, Gd₃Ga₂(GaO₄)₃: Cr³⁺, Y₃Sc₂(GaO₄)₃:Cr³⁺, La₃Sc₂(GaO₄)₃:Cr³⁺, Gd₃Sc₂(GaO₄)₃:Cr³⁺, and (Gd, La)₃(Ga, Sc)₂(GaO₄)₃:Cr³⁺. The Cr³⁺ activated garnet phosphor may be a solid solution having a phosphor described above as an end component.

The near-infrared phosphor included in the wavelength converter 2 may be at least one selected from the group consisting of Y₄CdMo₃O₁₆ : Yb³⁺, K₃LuSi₂O₇ : Eu²⁺, CaO : Eu²⁺, Sr_{2-y}Ca_{y}InSbO₆ : Fe³⁺, La₃Ga₅GeO₁₄ : Cr³⁺, ScBO₃ : Cr³⁺, and Ga₂₋ₓScₓO₃ : Cr³⁺.

Here, a spectral distribution of near-infrared light emitted from the near-infrared phosphor is preferably unimodal when spectral intensity for each wavelength of 10 nm is displayed in a histogram. Here, the term "unimodal" as used herein means a distribution state where the histogram has only one peak. By making the spectral distribution unimodal, near-infrared optical components are concentrated in a specific wavelength range, and thus the near-infrared light emitting device is advantageous for high output and high efficiency of near-infrared light.

The wavelength converter 2 can be manufactured by sealing a phosphor with a sealing material. The sealing material is preferably at least one of an organic material or an inorganic material, particularly at least one of a transparent (translucent) organic material or a transparent (translucent) inorganic material. An example of the sealing material of the organic material is a transparent organic material such as a silicone resin. An example of the sealing material of the inorganic material is a transparent inorganic material such as low melting point glass.

As the wavelength converter 2, a sintered body formed by sintering a phosphor and having multiple voids therein can be used. As the wavelength converter 2, a ceramic body formed by sintering a phosphor and not having multiple voids therein can be used. Since the wavelength converter 2 is such a sintered body or a ceramic body, the manufacturing and handling of the wavelength converter 2 become easy, and thus the wavelength converter is suitable for industrial production. Since fluorescent ions are distributed densely in the thickness direction due to such a sintered body or a ceramic body, the light absorption rate of the wavelength converter can be relatively easily increased, and the output ratio of fluorescence can be further increased.

The near-infrared light emitting device 10 is provided with the optical member 3, and the optical member 3 is arranged at a side of a surface (upper surface) of the wavelength converter 2, opposite the solid-state light emitting element 1. As illustrated in Fig. 3(a), the optical member 3 covers the entire upper surface of the wavelength converter 2. The optical member 3 has a property of attenuating part of fluorescence emitted by the near-infrared phosphor included in the wavelength converter 2, but transmitting other fluorescence.

In the near-infrared light emitting device 10, the optical member 3 is preferably made from only an inorganic material. Since the optical member 3 made from only an inorganic material is excellent in terms of heat resistance and durability, it is possible to obtain a near-infrared light emitting device having improved heat resistance and durability.

The optical member 3 preferably has a dielectric multilayer film structure. This makes it possible to obtain an optical property of attenuating part of fluorescence emitted from the near-infrared phosphor, but transmitting other fluorescence, by utilizing an optical interference effect observed in the multilayer structure of dielectrics having different permittivities.

The optical member 3 is preferably an optical interference filter. The optical interference filter is made by forming a dielectric thin film on the surface of a substrate. The dielectric thin film utilizes light transmission property changes due to interference of reflections occurring at interfaces between air and a dielectric, between a dielectric and a substrate, and between different dielectrics. By using such an optical interference filter, it becomes possible to lower the transmittance of part of fluorescence emitted by the near-infrared phosphor, and to relatively increase the transmittance of other fluorescence. Since the optical interference filter is easy to change the design and obtain, it becomes possible to configure a desired light emitting device relatively easily.

The optical member 3 is more preferably a notch filter. The notch filter is also called a band-stop filter or a band-prevention filter. The notch filter has a property of attenuating light in a specific wavelength band (stop band, blocking region) to a very low level, and transmitting light in most other wavelengths with a small intensity loss. By using a notch filter as the optical member 3, when a measurement object is irradiated with near-infrared fluorescence, it becomes possible to exclude unnecessary light in wavelengths other than two wavelengths.

The operation of the near-infrared light emitting device 10 according to the present embodiment having such a configuration will be described. As illustrated in Fig. 3(a), in the near-infrared light emitting device 10, when power is applied to the solid-state light emitting element 1 of the near-infrared light emitting device 10, primary light is emitted upward from the solid-state light emitting element 1. The emitted primary light transmits through the wavelength converter 2, and at this time, part of the primary light is absorbed by fluorescent ions of the near-infrared phosphor included in the wavelength converter 2. Then, it is converted into near-infrared light as wavelength-converted light through electron energy transition of fluorescent ions, and emitted from the phosphor.

As illustrated in Fig. 3(c), the near-infrared fluorescence emitted from the near-infrared phosphor has a broad spectral distribution with a large half width, and is preferably unimodal.

The near-infrared light emitted from the wavelength converter 2 reaches the optical member 3. As described above, the optical member 3 has a property of attenuating part of the near-infrared light, and transmitting other fluorescence. Thus, the near-infrared fluorescence entering from one surface 3a of the optical member 3 is partially attenuated by the optical member 3, and other fluorescence transmits therethrough. Then, near-infrared light having a spectral distribution as illustrated in Fig. 3(b) is output from the other surface 3b of the optical member 3. In this manner, the near-infrared light emitting device 10 can output output light where unimodal near-infrared fluorescence emitted from the wavelength converter 2 is partially attenuated.

Here, output light emitted from the near-infrared light emitting device 10 according to the present embodiment will be described in detail.

Since the near-infrared light emitting device 10 emits output light from the other surface 3b of the optical member 3, the fluorescent component is emitted from one light output surface. Consequently, the near-infrared light emitting device 10 has one optical axis of fluorescent components.

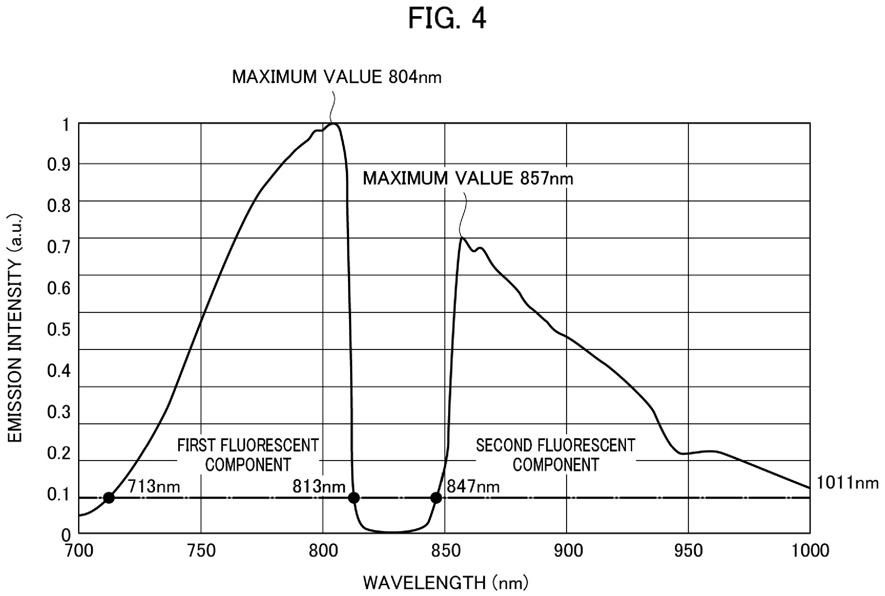

Since part of the near-infrared light emitted from the wavelength converter 2 is cut by the optical member 3 as described above, the output light has at least two fluorescent components as illustrated in Fig. 4. The two fluorescent components are a first fluorescent component, and a second fluorescent component positioned in order from the short wavelength side. Note that since the near-infrared phosphor includes a transition metal as an activator, the first fluorescent component and the second fluorescent component are based on electron energy transition of transition metal ions.

In the spectral distribution illustrated in Fig. 4, the first fluorescent component has a fluorescence peak wavelength of 804 nm, and the second fluorescent component has a fluorescence peak wavelength of 857 nm.

In the spectral shape of the first fluorescent component, a slope in the long wavelength region from the fluorescence peak of the first fluorescent component is steeper than that in the short wavelength region. Specifically, the short wavelength end of the first fluorescent component is set at a wavelength of 10% of the maximum emission intensity at the fluorescence peak of the first fluorescent component and at the fluorescence peak of the second fluorescent component, and the long wavelength end of the first fluorescent component is set at a wavelength of 10% of the maximum emission intensity. In detail, the short wavelength end of the first fluorescent component is set at a wavelength of 10% of the intensity at the fluorescence peak of the first fluorescent component, that is, at a wavelength of 713 nm, and the long wavelength end of the first fluorescent component is set at a wavelength of 10% of the intensity at the fluorescence peak of the first fluorescent component, that is, at a wavelength of 813 nm. As illustrated in Fig. 4, on the short wavelength side of the fluorescence peak of the first fluorescent component, the emission intensity continuously increases from a wavelength of 713 nm to a wavelength of 804 nm, and the slope is gentle. On the other hand, on the long wavelength side of the fluorescence peak of the first fluorescent component, the emission intensity continuously decreases from a wavelength of 804 nm to a wavelength of 813 nm, and the slope is steep.

In contrast, in the spectral shape of the second fluorescent component, a slope in the short wavelength region from the fluorescence peak of the second fluorescent component is steeper than that in the long wavelength region. Specifically, the short wavelength end of the second fluorescent component is set at a wavelength of 10% of the maximum emission intensity at the fluorescence peak of the first fluorescent component and at the fluorescence peak of the second fluorescent component, and the long wavelength end of the second fluorescent component is set at a wavelength of 10% of the maximum emission intensity. In detail, the short wavelength end of the second fluorescent component is set at a wavelength of 10% of the intensity at the fluorescence peak of the first fluorescent component, that is, at a wavelength of 847 nm, and the long wavelength end of the second fluorescent component is set at a wavelength of 10% of the intensity at the fluorescence peak of the first fluorescent component, that is, at a wavelength of 1,011 nm. As illustrated in Fig. 4, on the short wavelength side of the fluorescence peak of the second fluorescent component, the emission intensity continuously increases from a wavelength of 847 nm to a wavelength of 857 nm, and the slope is steep. On the other hand, on the long wavelength side of the fluorescence peak of the second fluorescent component, the emission intensity continuously decreases from a wavelength of 857 nm to a wavelength of 1,011 nm, and the slope is gentle.

The emission intensity between the long wavelength end of the first fluorescent component, and the short wavelength end of the second fluorescent component is 10% or less of the maximum emission intensity at the fluorescence peak of the first fluorescent component and at the fluorescence peak of the second fluorescent component.

As described above, in the near-infrared light emitting device 10 according to the present embodiment, the first fluorescent component and the second fluorescent component are clearly separated. The emission intensity between the first fluorescent component and the second fluorescent component is less than 10% of the maximum emission intensity at the fluorescence peak of the first fluorescent component and at the fluorescence peak of the second fluorescent component. Thus, for example, a target substance can be quantified with the two wavelength method using a fluorescent component at a wavelength of around 770 nm, and a fluorescent component at a wavelength of around 880 nm. Furthermore, the fluorescent component between the first fluorescent component and the second fluorescent component, which is not used in quantification using the two wavelength method, is largely cut, and thus adverse effects, such as discomfort to a measurement object, can be controlled.

The first fluorescent component emitted by the near-infrared light emitting device 10 will be described in more detail. In the spectral shape of the first fluorescent component, the maximum value of the slope in the long wavelength region from the fluorescence peak of the first fluorescent component is preferably 8% / nm or more when the maximum emission intensity at the fluorescence peak of the first fluorescent component and at the fluorescence peak of the second fluorescent component is 100%. That is, the maximum value of the slope in the long wavelength region from the fluorescence peak of the first fluorescent component is preferably 8% or more per 1 nm wavelength when the maximum emission intensity is 100%. On the other hand, the maximum value of the slope in the short wavelength region from the fluorescence peak of the first fluorescent component is preferably less than 8% / nm when the maximum emission intensity is 100%. That is, the maximum value of the slope in the short wavelength region from the fluorescence peak of the first fluorescent component is preferably less than 8% per 1 nm wavelength when the maximum emission intensity is 100%. Such a spectral distribution reduces fluorescent components between the first fluorescent component and the second fluorescent component, and increases the intensity of measurement light on the short wavelength side in the two wavelength method, and thus measurement accuracy of a target substance can be enhanced while controlling adverse effects on the measurement object.

Note that in the spectral distribution of the first fluorescent component, the maximum value of the slope in the long wavelength region from the fluorescence peak of the first fluorescent component is preferably 10% / nm or more when the maximum emission intensity at the fluorescence peak of the first fluorescent component and at the fluorescence peak of the second fluorescent component is 100%. The maximum value of the slope in the short wavelength region from the fluorescence peak of the first fluorescent component is preferably less than 5% / nm when the maximum emission intensity is 100%.

The second fluorescent component emitted by the near-infrared light emitting device 10 will be described in more detail. In the spectral shape of the second fluorescent component, the maximum value of the slope in the short wavelength region from the fluorescence peak of the second fluorescent component is preferably 8% / nm or more when the maximum emission intensity at the fluorescence peak of the first fluorescent component and at the fluorescence peak of the second fluorescent component is 100%. That is, the maximum value of the slope in the short wavelength region from the fluorescence peak of the second fluorescent component is preferably 8% or more per 1 nm wavelength when the maximum emission intensity is 100%. On the other hand, the maximum value of the slope in the long wavelength region from the fluorescence peak of the second fluorescent component is preferably less than 8% / nm when the maximum emission intensity is 100%. That is, the maximum value of the slope in the long wavelength region from the fluorescence peak of the second fluorescent component is preferably less than 8% per 1 nm wavelength when the maximum emission intensity is 100%. Such spectral distribution reduces fluorescent components between the first fluorescent component and the second fluorescent component, and increases the intensity of measurement light on the long wavelength side in the two wavelength method, and thus measurement accuracy of a target substance can be enhanced while controlling adverse effects on the measurement object.

Note that in the spectral distribution of the second fluorescent component, the maximum value of the slope in the short wavelength region from the fluorescence peak of the second fluorescent component is preferably 10%/nm or more when the maximum emission intensity at the fluorescence peak of the first fluorescent component and at the fluorescence peak of the second fluorescent component is 100%. The maximum value of the slope in the long wavelength region from the fluorescence peak of the second fluorescent component is preferably less than 5% / nm when the maximum emission intensity is 100%.

The short wavelength end of the first fluorescent component is set at a wavelength of 10% of the maximum emission intensity at the fluorescence peak of the first fluorescent component and at the fluorescence peak of the second fluorescent component, and the long wavelength end of the first fluorescent component is set at a wavelength of 10% of the maximum emission intensity. On the short wavelength side of the first fluorescent component, a wavelength range from the short wavelength end of the first fluorescent component to the fluorescence peak of the first fluorescent component is preferably 40 nm or more. On the long wavelength side of the first fluorescent component, a wavelength range from the fluorescence peak of the first fluorescent component to the long wavelength end of the first fluorescent component is preferably 15 nm or less. Such spectral distribution also reduces fluorescent components between the first fluorescent component and the second fluorescent component, and increases the intensity of measurement light on the short wavelength side in the two wavelength method, and thus measurement accuracy of a target substance can be enhanced while controlling adverse effects on the measurement object.

The short wavelength end of the second fluorescent component is set at a wavelength of 10% of the maximum emission intensity at the fluorescence peak of the first fluorescent component and at the fluorescence peak of the second fluorescent component, and the long wavelength end of the second fluorescent component is set at a wavelength of 10% of the maximum emission intensity. On the short wavelength side of the second fluorescent component, a wavelength range from the short wavelength end of the second fluorescent component to the fluorescence peak of the second fluorescent component is preferably 15 nm or less. On the long wavelength side of the second fluorescent component, a wavelength range from the fluorescence peak of the second fluorescent component to the long wavelength end of the second fluorescent component is preferably 40 nm or more. Such spectral distribution also reduces fluorescent components between the first fluorescent component and the second fluorescent component, and increases the intensity of measurement light on the long wavelength side in the two wavelength method, and thus measurement accuracy of a target substance can be enhanced while controlling adverse effects on the measurement object.

In the near-infrared light emitting device 10, the second fluorescent component preferably has a fluorescence peak within a wavelength range of 780 nm or more and 1,000 nm or less. Since the fluorescence peak of the second fluorescent component is within this wavelength range, measurement light on the long wavelength side can be near-infrared light.

In the near-infrared light emitting device 10, the first fluorescent component is positioned on the short wavelength side than the second fluorescent component, and thus includes at least one of red light or near-infrared light. Thus, the first fluorescent component preferably has a fluorescence peak within a wavelength range of 600 nm or more and 900 nm or less. Since the fluorescence peak of the first fluorescent component is within this wavelength range, measurement light on the short wavelength side can be at least one of red light or near-infrared light.

In the near-infrared light emitting device 10, the first fluorescent component and the second fluorescent component are based on electron energy transition of the same transition metal ion, and are preferably based on d-d transition or f-d transition. As described above, the near-infrared fluorescence emitted from the near-infrared phosphor of the wavelength converter 2 has a broad spectral distribution having a large half width, and is preferably unimodal. Thus, the near-infrared phosphor includes a transition metal ion as an activator, and fluorescence emitted from the near-infrared phosphor is fluorescence based on electron energy transition of the transition metal ion, and is preferably fluorescence due to d-d transition or f-d transition. Note that when fluorescence emitted from the transition metal ion is fluorescence due to f-f transition, the fluorescence has a small half width and a sharp spectral distribution. Thus, the fluorescence emitted from the transition metal ion is preferably not fluorescence due to f-f transition.

In the near-infrared light emitting device 10, a wavelength difference between the wavelength of the long wavelength end of the first fluorescent component, and the wavelength of the short wavelength end of the second fluorescent component is preferably within a range of 20 nm or more and 100 nm or less. The long wavelength end of the first fluorescent component is set at a wavelength of 10% of the maximum emission intensity at the fluorescence peak of the first fluorescent component and at the fluorescence peak of the second fluorescent component. The short wavelength end of the second fluorescent component is set at a wavelength of 10% of the maximum emission intensity. The wavelength difference between the wavelength of the long wavelength end of the first fluorescent component, and the wavelength of the short wavelength end of the second fluorescent component is preferably within the above range. Thus, the first fluorescent component and the second fluorescent component are clearly separated, and can be suitably used for the two wavelength method.

In the near-infrared light emitting device 10, two or more fluorescent components are preferably emitted from the same phosphor. That is, the first fluorescent component and the second fluorescent component are preferably due to light emission from the same phosphor. This facilitates obtaining fluorescence having a spectral distribution as illustrated in Fig. 4, and can be suitably used for the two wavelength method.

In the near-infrared light emitting device 10, the first fluorescent component preferably includes an optical component within a wavelength range of 600 nm or more and less than 900 nm. When the first fluorescent component includes a fluorescent component within this range, measurement light on the short wavelength side can be at least one of red light or near-infrared light.

In the near-infrared light emitting device 10, the first fluorescent component and the second fluorescent component are based on electron energy transition of a transition metal ion, and the transition metal ion is preferably at least one selected from the group consisting of Cr³⁺, Cr⁴⁺, Fe³⁺, and Eu²⁺. In this case, near-infrared fluorescence emitted from a near-infrared phosphor of the wavelength converter 2 has a broad spectral distribution with a large half width, and is likely to be unimodal. Thus, the fluorescence having the spectral distribution illustrated in Fig. 4 can be easily obtained, and can be suitably used for the two wavelength method.

Note that in the above description, the emission intensity at the long wavelength end of the first fluorescent component is 10% of the maximum emission intensity at the fluorescence peak of the first fluorescent component and at the fluorescence peak of the second fluorescent component, and the emission intensity at the short wavelength end of the first fluorescent component is also 10% of the maximum emission intensity. Similarly, the emission intensity at the long wavelength end of the second fluorescent component is 10% of the maximum emission intensity, and the emission intensity at the short wavelength end of the second fluorescent component is also 10% of the maximum emission intensity. However, the emission intensity at the long wavelength end of the first fluorescent component may be 5% of the maximum emission intensity at the fluorescence peak of the first fluorescent component and at the fluorescence peak of the second fluorescent component, and the emission intensity at the short wavelength end of the first fluorescent component may also be 5% of the maximum emission intensity. Similarly, the emission intensity at the long wavelength end of the second fluorescent component may be 5% of the maximum emission intensity, and the emission intensity at the short wavelength end of the second fluorescent component may also be 5% of the maximum emission intensity. The emission intensity at the long wavelength end of the first fluorescent component may be 3% of the maximum emission intensity at the fluorescence peak of the first fluorescent component and at the fluorescence peak of the second fluorescent component, and the emission intensity at the short wavelength end of the first fluorescent component may also be 3% of the maximum emission intensity. Similarly, the emission intensity at the long wavelength end of the second fluorescent component may be 3% of the maximum emission intensity, and the emission intensity at the short wavelength end of the second fluorescent component may also be 3% of the maximum emission intensity.

The emission intensity between the long wavelength end of the first fluorescent component, and the short wavelength end of the second fluorescent component is 10% or less of the maximum emission intensity at the fluorescence peak of the first fluorescent component and at the fluorescence peak of the second fluorescent component, as a whole. However, the emission intensity between the long wavelength end of the first fluorescent component, and the short wavelength end of the second fluorescent component may be 5% or less, or 3% or less, of the maximum emission intensity, as a whole.

In the fluorescent components emitted by the near-infrared light emitting device 10, the emission intensity of the fluorescence peak of the first fluorescent component may be larger than that of the fluorescence peak of the second fluorescent component. Conversely, the emission intensity of the fluorescence peak of the first fluorescent component may be smaller than the emission intensity of the fluorescence peak of the second fluorescent component. The difference between the emission intensity at the fluorescence peak of the first fluorescent component, and the emission intensity at the fluorescence peak of the second fluorescent component is preferably 50% or less, more preferably 30% or less, and more preferably 10% or less. Since the difference between the emission intensity at the fluorescence peak of the first fluorescent component, and the emission intensity at the fluorescence peak of the second fluorescent component is small, the difference in intensities of two types of measurement light used in a two component method is easily reduced, and measurement accuracy of a target substance can be improved.

When a target substance is quantitatively analyzed with the two wavelength method using the near-infrared light emitting device 10, wavelengths of measurement light used may be, for example, near 770 nm and near 880 nm. However, the wavelengths of the measurement light are not limited to such an aspect, and may be near 610 nm and near 880 nm. The wavelengths of the measurement light may be near 675 nm and near 840 nm. The wavelength of the measurement light may be near 630 nm and near 850 nm. The wavelengths of the measurement light may be near 660 nm and near 850 nm. The wavelengths of the measurement light may be near 780 nm and near 905 nm.

As described above, the near-infrared light emitting device 10 according to the present embodiment is a near-infrared light emitting device that includes the solid-state light emitting element 1, and the wavelength converter 2 including a phosphor, and emits two or more fluorescent components from one light output surface. The two or more fluorescent components include the first fluorescent component and the second fluorescent component which are positioned in order from the short wavelength side, and the first fluorescent component and the second fluorescent component are based on electron energy transition of a transition metal ion. In the spectral shape of the first fluorescent component, the slope in the long wavelength region from the fluorescence peak of the first fluorescent component is steeper than that in the short wavelength region, and in the spectral shape of the second fluorescent component, the slope in the short wavelength region from the fluorescence peak of the second fluorescent component is steeper than that in the long wavelength region. The emission intensity at the long wavelength end of the first fluorescent component is 10% of the maximum emission intensity at the fluorescence peak of the first fluorescent component and at the fluorescence peak of the second fluorescent component, and the emission intensity at the short wavelength end of the second fluorescent component is 10% of the maximum emission intensity. The second fluorescent component has a fluorescence peak within a wavelength range of 780 nm or more and 1,000 nm or less.

The near-infrared light emitting device 10 according to the present embodiment is a near-infrared light emitting device that includes the solid-state light emitting element 1, and the wavelength converter 2 including a phosphor, and emits two or more fluorescent components from one light output surface. The near-infrared light emitting device 10 further includes a notch filter that controls wavelength-converted light converted by a wavelength converter. The fluorescent components have a first fluorescent component on the short wavelength side, and a second fluorescent component on the long wavelength side across a blocking region controlled by the notch filter. The first fluorescent component and the second fluorescent component are based on electron energy transition of a transition metal ion. The second fluorescent component has the fluorescence peak within a wavelength range of 780 nm or more and 1,000 nm or less.

Since the near-infrared light emitting device 10 emits fluorescent components from one light output surface, the fluorescent components have one axis, and thus the intensity balance is constant at any location on a measurement object. Output light emitted from the near-infrared light emitting device 10 has two fluorescent components, and the spectral distribution of the fluorescent components has a characteristic shape as illustrated in Fig. 4. Thus, fluorescent components between the first fluorescent component and the second fluorescent component are largely cut while a target substance is accurately quantified using the two wavelength method, and thus adverse effects, such as discomfort to the measurement object, can be controlled.

Note that the near-infrared light emitting device 10 according to the present embodiment is not limited to the configuration including the optical member illustrated in Fig. 3(a), and any configuration can be applied as long as it emits output light having the characteristic spectral distribution described above. In addition, the spectral distribution of the output light emitted from the near-infrared light emitting device 10 according to the present embodiment is not limited to the spectral distribution illustrated in Fig. 4.

### [Biological information detection device]

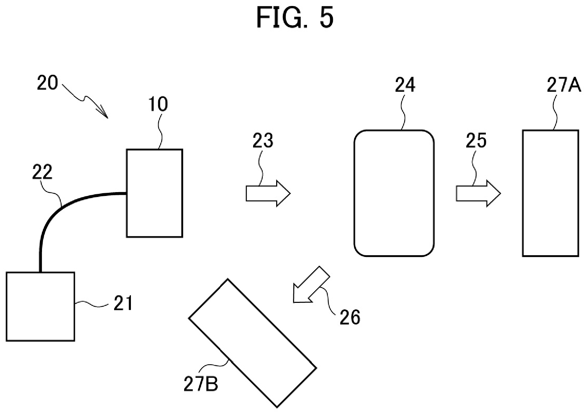

Next, a biological information detection device according to the present embodiment will be described. The biological information detection device according to the present embodiment includes the near-infrared light emitting device 10 described above. Fig. 5 schematically illustrates an example of the biological information detection device according to the present embodiment. The biological information detection device 20 at least includes a power supply circuit 21, a conductor 22, and the near-infrared light emitting device 10. The power supply circuit 21 supplies power to the solid-state light emitting element 1 in the near-infrared light emitting device 10 through the conductor 22.

As described above, the near-infrared light emitting device 10 converts electric energy into light energy. The near-infrared light emitting device 10 converts at least part of electric energy supplied from the power supply circuit 21 into light energy that becomes output light 23, and outputs it. Note that the near-infrared light emitting device 10 in Fig. 5 is configured to emit the output light 23, which includes at least near-infrared light. Note that the near-infrared light emitting device 10 may be configured to emit the output light 23, which includes red light and near-infrared light.

The biological information detection device 20 in Fig. 5 further includes a first detector 27A and a second detector 27B. The first detector 27A detects a transmitted optical component 25 of the output light 23, which has been emitted from the near-infrared light emitting device 10 on an object to be irradiated 24. Specifically, the first detector 27A detects near-infrared light in the transmitted optical component 25, which has transmitted through the object to be irradiated 24. The second detector 27B detects a reflected optical component 26 in the output light 23, which has been emitted from the near-infrared light emitting device 10 on the object to be irradiated 24. Specifically, the second detector 27B detects near-infrared light in the reflected optical component 26, which has been reflected by the object to be irradiated 24. Note that the first detector 27A and the second detector 27B may detect red light in addition to near-infrared light.

In the biological information detection device 20 having such a configuration, the object to be irradiated 24, which is a measurement object, is irradiated with the output light 23 including near-infrared light, and the transmitted optical component 25, which has transmitted through the object to be irradiated 24, and the reflected optical component 26, which has been reflected by the object to be irradiated 24, are detected by the first detector 27A and the second detector 27B. Thus, it becomes possible for the biological information detection device 20 to detect property information of the object to be irradiated 24, where a near-infrared optical component is involved.

Here, the near-infrared light emitting device 10 according to the present embodiment can emit the output light 23, which includes at least near-infrared light and is convenient for detectors. Thus, combination of the light emitting device and a near-infrared detector provides a biological information detection device suitable for industrial use.

Various types of photodetectors can be used for the first detector 27A and the second detector 27B. Specifically, a quantum-type photodetector (photodiode, phototransistor, photo IC, CCD image sensor, CMOS image sensor, and the like) that detects a charge generated when light enters a PN junction of a semiconductor can be used depending on the mode of use of the biological information detection device. The photodetector can also be a heat-type photodetector (thermopile using thermoelectric effect, pyroelectric element using pyroelectric effect, and the like) that detects a change in electrical properties caused by a temperature rise due to heat generated when light is received, or an infrared film that is sensitive to light.

As the first detector 27A and the second detector 27B, a single element utilizing a photoelectric conversion element alone may be used, or an imaging element integrating photoelectric conversion elements may be used. The form of the imaging element may be linear with a one-dimensional arrangement, or planar with a two-dimensional arrangement. An imaging camera can also be used as the first detector 27A and the second detector 27B.

Note that although the biological information detection device 20 in Fig. 5 includes both the first detector 27A and the second detector 27B, the biological information detection device only needs to include at least one of the first detector 27A or the second detector 27B.

The biological information detection device according to the present embodiment can be used for medical, veterinary, and biotechnological purposes. The biological information detection device according to the present embodiment can be used for any of the human body, animals, and plants, and can be used for any of gases, liquids, and solids.

The biological information detection device according to the present embodiment is preferably used as a medical device, a therapeutic device, a beauty device, a health device, a care related device, an analytical device, a measuring device, or an evaluation device.

For example, for the purpose of medical and biotechnique development, the biological information detection device according to the present embodiment can be used for examination, detection, measurement, evaluation, assay, analysis, observation, monitoring, separation, diagnosis, treatment, purification, and the like of 1) blood, body fluid, and their components, 2) excreta (urine and feces), 3) proteins and amino acids, 4) cells (including cancer cells), 5) genes, chromosomes, and nucleic acids, 6) biological samples, bacteria, specimens, and antibodies, 7) biological tissues, organs, and blood vessels, and 8) skin diseases and alopecia.

For example, for the purpose of beauty and health care, the biological information detection device according to the present embodiment can be used for examination, detection, measurement, evaluation, assay, analysis, observation, monitoring, beautification, hygiene, growth promotion, health promotion, diagnosis, and the like of 1) skin, 2) hair and body hair, 3) oral, endodontic, and periodontal conditions, 4) ear and nose, and 5) vital signs.

For example, for the purpose of nursing care, the biological information detection device according to the present embodiment can be used to check excretion and to identify, manage, monitor, and the like of health conditions.

### (Note)

The description of the above embodiment discloses the following techniques.
(Technique 1) A near-infrared light emitting device including:
a solid-state light emitting element; and
a wavelength converter that includes a phosphor, wherein
the near-infrared light emitting device emits two or more fluorescent components from one light output surface,
the two or more fluorescent components include a first fluorescent component and a second fluorescent component positioned in order from a short wavelength side, the first fluorescent component and the second fluorescent component being based on electron energy transition of a transition metal ion,
in a spectral shape of the first fluorescent component, a slope in a long wavelength region from a fluorescence peak of the first fluorescent component is steeper than that in a short wavelength region, and in a spectral shape of the second fluorescent component, a slope in a short wavelength region from a fluorescence peak of the second fluorescent component is steeper than that in a long wavelength region,
an emission intensity of the first fluorescent component at a long wavelength end is 10% of a maximum emission intensity at the fluorescence peak of the first fluorescent component and at the fluorescence peak of the second fluorescent component, and an emission intensity of the second fluorescent component at a short wavelength end is 10% of the maximum emission intensity, and
the second fluorescent component has the fluorescence peak, which is within a wavelength range of 780 nm or more and 1,000 nm or less.

With this configuration, the intensity balance of measurement light is constant at any place of a measuring object. Furthermore, since fluorescent components between the first fluorescent component and the second fluorescent component are largely cut while a target substance is accurately quantified using the two wavelength method, adverse effects, such as discomfort to the measuring object, can be controlled.
(Technique 2) A near-infrared light emitting device including:
a solid-state light emitting element; and
a wavelength converter that includes a phosphor, wherein
the near-infrared light emitting device emits two or more fluorescent components from one light output surface,
the near-infrared light emitting device further includes a notch filter for controlling wavelength-converted light converted by the wavelength converter,
the fluorescent components include a first fluorescent component on a short wavelength side, and a second fluorescent component on a long wavelength side across a blocking region controlled by the notch filter, the first fluorescent component and the second fluorescent component being based on electron energy transition of a transition metal ion, and
the second fluorescent component has a fluorescence peak within a wavelength range of 780 nm or more and 1,000 nm or less.

With this configuration, the intensity balance of measurement light is constant at any place of a measuring object. Furthermore, since fluorescent components between the first fluorescent component and the second fluorescent component are largely cut while a target substance is accurately quantified using the two wavelength method, adverse effects, such as discomfort to the measuring object, can be controlled.
(Technique 3) The near-infrared light emitting device according to technique 1 or 2, wherein
in a spectral shape of the first fluorescent component, a maximum value of the slope in the long wavelength region from the fluorescence peak of the first fluorescent component is 8% / nm or more when a maximum emission intensity in the fluorescence peak of the first fluorescent component and at the fluorescence peak of the second fluorescent component is 100%, and a maximum value of the slope in the short wavelength region from the fluorescence peak of the first fluorescent component is less than 8% / nm, when the maximum emission intensity is 100%;
in a spectral shape of the second fluorescent component, a maximum value of the slope in the short wavelength region from the fluorescence peak of the second fluorescent component is 8% / nm or more when the maximum emission intensity is 100%, and a maximum value of the slope in the long wavelength region from the fluorescence peak of the second fluorescent component is less than 8% / nm, when the maximum emission intensity is 100%.

With this configuration, since the intensity of two types of measurement light increases while fluorescent components between the first fluorescent component and the second fluorescent component are cut, measurement accuracy of a target substance can be improved while adverse effects on the measurement object are controlled.
(Technique 4) The near-infrared light emitting device according to any one of techniques 1 to 3, wherein the first fluorescent component and the second fluorescent component are based on electron energy transition of a same transition metal ion, and are based on d-d transition or f-d transition.

With this configuration, near-infrared fluorescence emitted from the wavelength converter has a broad spectral distribution with a large half width, and thus it is possible to easily obtain the first fluorescent component and the second fluorescent component with a characteristic spectral distribution.
(Technique 5) The near-infrared light emitting device according to any one of techniques 1 to 4, wherein a wavelength difference between a wavelength of the long wavelength end of the first fluorescent component, and a wavelength of the short wavelength end of the second fluorescent component is within a range from 20 nm or more and 100 nm or less.

With this configuration, the first fluorescent component and the second fluorescent component are clearly separated, and thus measurement accuracy of a target substance using the two wavelength method can be further enhanced.
(Technique 6) The near-infrared light emitting device according to any one of techniques 1 to 5, wherein the two or more fluorescent components are emissions from the same phosphor.

With this configuration, fluorescence having a characteristic spectral distribution including the first fluorescent component and the second fluorescent component can be easily obtained, and thus can be suitably used for the two wavelength method.
(Technique 7) The near-infrared light emitting device according to any one of techniques 1 to 6, wherein the first fluorescent component includes an optical component within a wavelength range of 600 nm or more and 900 nm or less.

With this configuration, measurement light on the short wavelength side can be at least one of red light or near-infrared light.
(Technique 8) The near-infrared light emitting device according to any one of techniques 1 to 7, wherein the transition metal ion is at least one selected from the group consisting of Cr³⁺, Cr⁴⁺, Fe³⁺, and Eu²⁺.

With this configuration, fluorescence having a characteristic spectral distribution including the first fluorescent component and the second fluorescent component can be easily obtained, and thus can be suitably used for the two wavelength method.
(Technique 9) A biological information detection device including the near-infrared light emitting device according to any one of techniques 1 to 8.

With this configuration, a biological information detection device can be obtained which is capable of accurately acquiring biological information using the two wavelength method.

### EXAMPLES

Hereinafter, the present embodiment will be described in more detail with reference to examples and comparative examples, but the present embodiment is not limited to these examples.

### [Manufacture of light emitting device]

First, fluorescent ceramics were synthesized using a preparation method using a solid-state reaction. Specifically, a fluorescent ceramic represented by the composition formula (Ga_{0.69}, Sc_{0.3}, Cr_{0.01})₂O₃ was synthesized. In addition, a fluorescent ceramic represented by the composition formula Gd₃(Ga_{0.97}, Cr_{0.03})₂Ga₃O₁₂ was synthesized.

When each of the fluorescent ceramics was synthesized, the following compound powders were used as main raw materials.
Gallium oxide (Ga₂O₃): purity 4N, manufactured by Nippon Rare Metal, Inc.
Scandium oxide (Sc₂O₃): purity 3N, manufactured by Kojundo Chemical Lab. Co., Ltd.
Chromium oxide (Cr₂O₃): purity 3N, manufactured by Kojundo Chemical Lab. Co., Ltd.
Boric acid (H₃BO₃): purity 2N, manufactured by FUJIFILM Wako Pure Chemical Corporation
Gadolinium oxide (Gd₂O₃): purity 4N, manufactured by Nippon Yttrium Co., Ltd.

First, raw materials of the above fluorescent ceramics were weighed under blending conditions in Table 1. Next, the weighed raw materials were put in a pot of a planetary ball mill, and after water and alumina balls of Φ3 mm were added, the raw materials were wet-mixed using the planetary ball mill. Then, a mixed slurry obtained was dried sufficiently in a constant temperature bath at 125 °C, and was lightly crushed using a mortar and pestle to obtain a raw material mixed powder.

Next, 1 g of the obtained raw material mixed powder of each example was added to a mold (Φ13 mm), and then pressed using a hand press at a pressure of 10 MPa to produce a molded body. Then, each of the molded bodies was calcined under calcination conditions listed in Table 2 to obtain a sintered compact.

Each of the sintered compacts manufactured was polished to a film thickness of 100 µm using an automatic grinder (product number: DAG810, manufactured by Disco Corporation) to obtain each fluorescent ceramic. Finally, each of the fluorescent ceramics was processed to a size of 3.2 mm × 2.6 mm using an automatic dicing saw (product number: DAD3350, manufactured by Disco Corporation).

**[Table 1]**

| Composition formula | Blending ratio | Raw material (g) | | | | |
|---|---|---|---|---|---|---|
| | | Gd₂O₃ | Ga₂O₃ | Sc₂O₃ | Cr₂O₃ | H₃BO₃ |
| (Ga_{0.69},Sc_{0.3},Cr_{0.01})₂O₃ | 0.69Ga₂O₃ · 0.30Sc₂O₃ · 0.01Cr₂O₃ · 0.03H₃BO₃ | 0 | 7.517 | 2.405 | 0.088 | 0.108 |
| Gd₃(Ga_{0.97},Cr_{0.03})₂Ga₃O₁₂ | 1.5Ga₂O₃ · 2.47Ga₂O₃ · 0.03Cr₂O₃ | 5.382 | 4.583 | 0 | 0.045 | 0 |

**[Table 2]**

| Composition formula | Calcination conditions | |
|---|---|---|
| | Calcination temperature | Calcination atmosphere |
| (Ga_{0.69},Sc_{0.3},Cr_{0.01})₂O₃ | 1500 °C | Atmosphere |
| Gd₃(Ga_{0.97},Cr_{0.03})₂Ga₃O₁₂ | 1600 °C | Nitrogen |

Next, a commercial blue LED was prepared. LEB P2MQ, product code LEB P2MQ-GSHQ-23-0, manufactured by ams-OSRAM AG was used as the blue LED. A light emitting module was obtained by stacking and placing a fluorescent ceramic described above on the blue LED.

Next, a notch filter was prepared as an optical member. An OD4.0 notch filter, product code #86-703, manufactured by Edmund Optics Inc. was used as the notch filter. The center wavelength of a blocking region of the notch filter was 830 nm, and the full width at half maximum was 42 nm. Reflectance in the blocking region was 99% or more. A transmission band other than the blocking region of the notch filter was within a range from 625 to 1,100 nm, and the transmittance is 90% or more.

The notch filter was mounted on the light emitting module to obtain a light emitting device of the present example.

### [Evaluation of spectral distribution]

The operation of the light emitting device of the example obtained as described above was confirmed. Specifically, when power (11.37 V, 500 mA) was applied to a blue LED chip of the light emitting device, blue light as primary light was emitted from the blue LED chip. Then, part of the light was absorbed by Cr³⁺ included in the fluorescent ceramic, and converted, through electron energy transition of Cr³⁺, into light that includes a near-infrared ray which is wavelength-converted light.

Since the wavelength-converted light was transmitted through the notch filter, light having a wavelength of around 830 nm was cut off, and the remaining light was output. The spectral distribution of the light output from the light emitting device of the present example is the spectral distribution illustrated in Fig. 4.

In the spectral distribution illustrated in Fig. 4, the fluorescence peak wavelength of the first fluorescent component is 804 nm, and the fluorescence peak wavelength of the second fluorescent component is 857 nm. The short wavelength end of the first fluorescent component is a wavelength of 10% of the intensity at the fluorescence peak of the first fluorescent component, that is, a wavelength of 713 nm, and the long wavelength end of the first fluorescent component is a wavelength of 10% of the intensity at the fluorescence peak of the first fluorescent component, that is, a wavelength of 813 nm. The short wavelength end of the second fluorescent component is a wavelength of 10% of the intensity at the fluorescence peak of the first fluorescent component, that is, a wavelength of 847 nm, and the long wavelength end of the second fluorescent component is a wavelength of 10% of the intensity at the fluorescence peak of the first fluorescent component, that is, a wavelength of 1,011 nm.

The maximum value of the slope in the long wavelength region from the fluorescence peak of the first fluorescent component was 25.86% / nm when the maximum emission intensity at the fluorescence peak of the first fluorescent component was 100%. The maximum value of the slope in the short wavelength region from the fluorescence peak of the first fluorescent component was 1.70% / nm when the maximum emission intensity was 100%.

The maximum value of the slope in the short wavelength region from the fluorescence peak of the second fluorescent component was 13.00% / nm when the maximum emission intensity at the first fluorescent component was 100%. The maximum value of the slope in the long wavelength region from the fluorescence peak of the second fluorescent component was 2.11% / nm when the maximum emission intensity was 100%.

Note that on the short wavelength side of the first fluorescent component, the wavelength range from the short wavelength end of the first fluorescent component to the fluorescence peak of the first fluorescent component was 91 nm. On the long wavelength side of the first fluorescent component, the wavelength range from the fluorescence peak of the first fluorescent component to the long wavelength end of the first fluorescent component was 9 nm.

On the short wavelength side of the second fluorescent component, the wavelength range from the short wavelength end of the second fluorescent component to the fluorescence peak of the second fluorescent component was 10 nm. On the long wavelength side of the second fluorescent component, the wavelength range from the fluorescence peak of the second fluorescent component to the long wavelength end of the second fluorescent component was 154 nm.

As described above, in the output light emitted from the light emitting device according to example, the slope in the long wavelength region from the fluorescence peak of the first fluorescent component was steeper than that in the short wavelength region, and the slope in the short wavelength region from the fluorescence peak of the second fluorescent component was steeper than that in the long wavelength region. Thus, while the target substance is accurately quantified using the two wavelength method, fluorescent components between the first fluorescent component and the second fluorescent component are largely cut, and thus adverse effects such as discomfort on the measurement object can be controlled.

Note that in Fig. 4, light having a wavelength of 930 nm or more is unnecessary light which is not used as measurement light. Thus, light having a wavelength of 930 nm or more may be cut by using, for example, a low-pass filter.

Although the present embodiment has been described above, the present embodiment is not limited to these descriptions, and various modifications are possible within the scope of the gist of the present embodiment.

The entire contents of Japanese Patent Application No. 2023-119019 (application date: July 21, 2023) are incorporated herein by reference.

### INDUSTRIAL APPLICABILITY

In the present disclosure, it is possible to provide a near-infrared light emitting device capable of controlling occurrence of unevenness in irradiation of light emitted on a measurement object even when light of two different wavelengths is emitted.

### REFERENCE SIGNS LIST

- 1: Solid-state light emitting element
- 2: Wavelength converter
- 3: Optical member (notch filter)
- 10: Near-infrared light emitting device

## Claims

1. A near-infrared light emitting device comprising:
a solid-state light emitting element; and
a wavelength converter that includes a phosphor, wherein
the near-infrared light emitting device emits two or more fluorescent components from one light output surface,
the two or more fluorescent components include a first fluorescent component and a second fluorescent component positioned in order from a short wavelength side, the first fluorescent component and the second fluorescent component being based on electron energy transition of a transition metal ion,
in a spectral shape of the first fluorescent component, a slope in a long wavelength region from a fluorescence peak of the first fluorescent component is steeper than that in a short wavelength region, and in a spectral shape of the second fluorescent component, a slope in a short wavelength region from a fluorescence peak of the second fluorescent component is steeper than that in a long wavelength region,
an emission intensity of the first fluorescent component at a long wavelength end is 10% of a maximum emission intensity at the fluorescence peak of the first fluorescent component and at the fluorescence peak of the second fluorescent component, and an emission intensity of the second fluorescent component at a short wavelength end is 10% of the maximum emission intensity, and
the second fluorescent component has the fluorescence peak, which is within a wavelength range of 780 nm or more and 1,000 nm or less.

2. A near-infrared light emitting device comprising:
a solid-state light emitting element; and
a wavelength converter that includes a phosphor, wherein
the near-infrared light emitting device emits two or more fluorescent components from one light output surface,
the near-infrared light emitting device further comprises a notch filter for controlling wavelength-converted light converted by the wavelength converter,
the fluorescent components include a first fluorescent component on a short wavelength side, and a second fluorescent component on a long wavelength side across a blocking region controlled by the notch filter, the first fluorescent component and the second fluorescent component being based on electron energy transition of a transition metal ion, and
the second fluorescent component has a fluorescence peak within a wavelength range of 780 nm or more and 1,000 nm or less.

3. The near-infrared light emitting device according to claim 1 or 2, wherein
in the spectral shape of the first fluorescent component, a maximum value of the slope in the long wavelength region from the fluorescence peak of the first fluorescent component is 8% / nm or more when the maximum emission intensity at the fluorescence peak of the first fluorescent component and at the fluorescence peak of the second fluorescent component is 100%, and a maximum value of the slope in the short wavelength region from the fluorescence peak of the first fluorescent component is less than 8% / nm, when the maximum emission intensity is 100%;
in the spectral shape of the second fluorescent component, a maximum value of the slope in the short wavelength region from the fluorescence peak of the second fluorescent component is 8% / nm or more when the maximum emission intensity is 100%, and a maximum value of the slope in the long wavelength region from the fluorescence peak of the second fluorescent component is less than 8% / nm, when the maximum emission intensity is 100%.

4. The near-infrared light emitting device according to any one of claims 1 to 3, wherein
the first fluorescent component and the second fluorescent component are based on electron energy transition of a same transition metal ion, and are based on d-d transition or f-d transition.

5. The near-infrared light emitting device according to claim 1, wherein a wavelength difference between a wavelength of the long wavelength end of the first fluorescent component, and a wavelength of the short wavelength end of the second fluorescent component is within a range from 20 nm or more and 100 nm or less.

6. The near-infrared light emitting device according to any one of claims 1 to 5, wherein the two or more fluorescent components are emissions from the same phosphor.

7. The near-infrared light emitting device according to any one of claims 1 to 6, wherein the first fluorescent component includes an optical component within a wavelength range of 600 nm or more and 900 nm or less.

8. The near-infrared light emitting device according to any one of claims 1 to 7, wherein the transition metal ion is at least one selected from the group consisting of Cr³⁺, Cr⁴⁺, Fe³⁺, and Eu²⁺.

9. A biological information detection device comprising:
the near-infrared light emitting device according to any one of claims 1 to 8.
